# EUROPEAN PATENT APPLICATION

(11) **EP 4 779 645 A1**
(43) Date of publication of application: **22.07.2026**
(21) Application number: 23956034.5
(22) Date of filing: 17.10.2023
(51) Int. Cl.: G16H 10/00, G16H 20/00

(54) **THERAPEUTIC APPLICATION MANAGEMENT SYSTEM AND MANAGEMENT SERVER**

(71) Applicant: Susmed, Inc., Tokyo 103-0023 (JP)
(72) Inventor: MOTOHASHI Tomomitsu, Tokyo 103-0023 (JP); TAKAJO Kentaro, Tokyo 103-0023 (JP); OKUMURA Kosuke, Tokyo 103-0023 (JP)
(74) Representative: Cabinet Chaillot
(86) International application number: PCT/JP2023/037535
(87) International publication number: WO 2025/083782

(57) **Abstract**

There are included: a code information transmission unit 21 that analyzes a code using a function of a therapeutic application installed on a patient terminal 2 and transmits, to a management server, medical institution information obtained thereby together with a therapeutic application ID; a medical-institution-side information recording unit 11 that receives medical institution information and a management application ID from a medical institution terminal 3 and records these **in** a storage unit 10; and an activation unit 13 that receives the medical institution information and the therapeutic application ID transmitted from the code information transmission unit 21 and records, **in** the storage unit 10, the management application ID associated with the received medical institution information in association with the received therapeutic application ID, thereby activating the therapeutic application. Therefore, the use of a therapeutic application prescribed to a patient can be started **in** a state associated with the medical institution without requiring a medical professional to input patient information via the medical institution terminal 3 for each patient to whom the therapeutic application is to be prescribed and to transmit the information to the management server 1.

## Description

### Technical Field

The present invention relates to a therapeutic application management system and a management server, and in particular, is suitable for use in a system that manages the activation of therapeutic applications prescribed to patients.

### Background Art

In recent years, "therapeutic applications" intended to improve user health-related behaviors, treat diseases, and manage physical conditions have been drawing attention and are being developed. The primary functions provided by therapeutic applications are to record the daily behaviors or symptoms of a patient and, based on the recorded information, to provide advice or messages indicating what the patient should do to improve their symptoms.

In general, therapeutic applications that are approved as medical equipment under the Pharmaceuticals and Medical Equipment Act should be prescribed by a doctor to a patient through a medical interview or the like and installed and used on a smartphone in accordance with the prescription. In this regard, a system is known in which a therapeutic application is set to a usable state using an activation code (see, for example, Patent Literature 1).

In the system described in Patent Literature 1, a server receives a request for an activation code from an information processing terminal of a user (patient) who is a subject of prescription of a therapeutic application, generates an activation code based on initial information regarding the patient's disease that has been entered in advance by a medical professional, and transmits the activation code to the patient's information processing terminal. On the patient's information processing terminal, the therapeutic application is executed in a state in which the initial information has been applied, using the activation code received from the server.

According to the technique described in Patent Literature 1, when a patient uses a therapeutic application for the first time, information specific to the patient is reflected in the therapeutic application. Therefore, even at the first use, the therapeutic application can be operated in a state suitable for the patient's medical condition. However, in order to realize this, it is necessary for a medical professional to input initial information regarding the patient's disease in advance to register the initial information in the server.

In a situation in which a medical professional have to input initial information each time a therapeutic application is prescribed to each individual patient, the burden on the medical professional becomes large. Although therapeutic applications are a new medical technology expected to become widespread in the future, when medical professionals are reluctant to introduce these due to the large burden on themselves, there is a risk that this will hinder the widespread adoption of therapeutic applications.

Note that a therapeutic application also provides functions for providing information recorded in the therapeutic application to a medical professional or for enabling a patient to communicate with the medical professional. For this purpose, it is necessary to register the therapeutic application used by each patient in association with a medical institution. However, Patent Literature 1 makes no mention of this matter.

### Citation List

### Patent Literature

PTL 1: JP2022-70086A

### Summary of Invention

### Technical Problem

The invention has been made to solve such problems, and an object thereof is to enable a therapeutic application prescribed to a patient to be started for use in a state associated with a medical institution, without imposing a significant burden on a medical professional.

### Solution to Problem

In order to solve the above-described problems, in the invention, in a management server that manages a therapeutic application installed on a patient terminal and a management application for transmitting and receiving information in cooperation with the therapeutic application, medical institution information related to a medical institution that uses the management application and identification information of the management application are recorded in a storage unit in association with each other, and a code in which the medical institution information is recorded is issued. In addition, the code in which the medical institution information is recorded is analyzed using a function of the therapeutic application installed on the patient terminal, and medical institution information obtained thereby is transmitted to the management server together with identification information of the therapeutic application. Then, in the management server, the medical institution information and the identification information of the therapeutic application are received from the patient terminal, and the identification information of the management application associated with the received medical institution information and the received identification information of the therapeutic application are recorded in the storage unit in association with each other, thereby activating the therapeutic application into a state in which information can be transmitted and received between the therapeutic application and the management application.

### Advantageous Effects of Invention

According to the invention configured as described above, the identification information of the management application stored in the storage unit together with the medical institution information is associated with the identification information of the therapeutic application transmitted to the management server together with the medical institution information obtained by analyzing the code with the therapeutic application, and the therapeutic application is activated into a state in which information can be transmitted and received between the therapeutic application and the management application. Therefore, a medical professional does not need to input patient information through the medical institution terminal for each patient to whom the therapeutic application is to be prescribed and transmit the patient information to the management server. As a result, according to the invention, the use of a therapeutic application prescribed to a patient can be started in a state associated with a medical institution without imposing a significant burden on a medical professional.

### Brief Description of Drawings

[FIG. 1] FIG. 1 is a diagram showing an example of the overall configuration of a therapeutic application management system according to a present embodiment.
[FIG. 2] FIG. 2 is a block diagram showing an example of the functional configuration of a therapeutic application management system according to the present embodiment.
[FIG. 3] FIG. 3 is a diagram schematically showing an example of information stored in a storage unit of the present embodiment.
[FIG. 4] FIG. 4 is a block diagram showing another example of the functional configuration of the therapeutic application management system according to the present embodiment.
[FIG. 5] FIG. 5 is a diagram showing another example of the overall configuration of the therapeutic application management system according to the present embodiment.
[FIG. 6] FIG. 6 is a block diagram showing another example of the functional configuration of the therapeutic application management system according to the present embodiment.

### Description of Embodiments

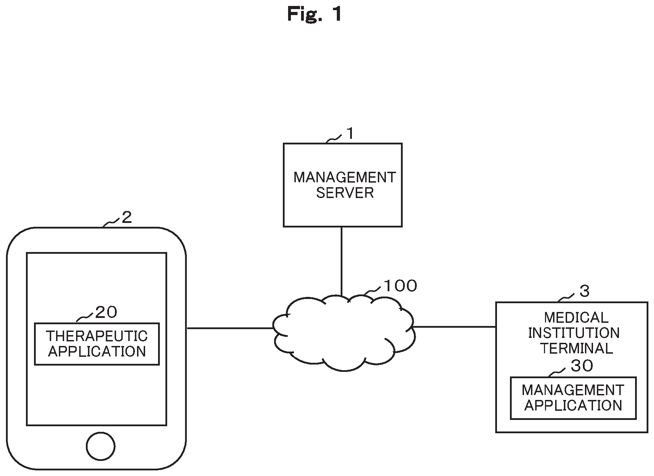

Hereinafter, an embodiment of the invention will be described with reference to the diagrams. FIG. 1 is a diagram showing an example of the overall configuration of a therapeutic application management system according to the present embodiment. As shown in FIG. 1, the therapeutic application management system according to the present embodiment includes a patient terminal 2 on which a therapeutic application 20 is installed, a medical institution terminal 3 on which a management application 30 for transmitting and receiving information in cooperation with the therapeutic application 20 is installed, and a management server 1 for managing the therapeutic application 20 and the management application 30.

The management server 1, the patient terminal 2, and the medical institution terminal 3 are connected to each other through a communication network 100 such as the Internet or a mobile phone network. Note that, although FIG. 1 shows one patient terminal 2 and one medical institution terminal 3 to simplify the illustration, in reality, there are as many patient terminals 2 as the number of patients using the therapeutic application 20, and there are as many medical institution terminals 3 as the number of medical institutions prescribing the therapeutic application 20.

The patient terminal 2 is a terminal used by a patient, and is, for example, a smartphone, a tablet, or a personal computer. The patient terminal 2 can download the therapeutic application 20 from a website connected to the communication network 100, install it, and use it. Note that, although the patient terminal 2 is assumed to be a smartphone, a tablet, or a personal computer herein, the patient terminal 2 is not limited thereto. That is, any terminal that can download and install the therapeutic application 20 can be used as the patient terminal 2.

The medical institution terminal 3 is a terminal used by a medical professional at a medical institution, and is, for example, a smartphone, a tablet, or a personal computer. The medical institution terminal 3 can download the management application 30 from a website connected to the communication network 100, install it, and use it. Note that, although the medical institution terminal 3 is assumed to be a smartphone, a tablet, or a personal computer herein, the medical institution terminal 3 is not limited thereto. That is, any terminal that can download and install the management application 30 can be used as the medical institution terminal 3.

Here, a configuration is shown in which the medical institution terminal 3 installs and uses the management application 30; however, the invention is not limited thereto. For example, the management application 30 may be configured as a web application, and the management application 30 may be used by accessing the operation server of the management application 30 from the web browser of the medical institution terminal 3 using a login ID. In this case, the operation server may be the management server 1 or another server having some of the functions of the management server 1.

The therapeutic application 20 installed on the patient terminal 2 and the management application 30 installed on the medical institution terminal 3 transmit and receive various kinds of information through the management server 1 connected to the communication network 100, and perform various processes related to improving the patient's symptoms. In this regard, the therapeutic application 20 has functions prepared according to the disease to be treated.

For example, the therapeutic application 20 has a function of transmitting various kinds of data to the management server 1, such as daily behavior information, biological information, symptom information, and questionnaire response information that are input or measured by the patient. Regarding this function, the management server 1 stores various kinds of data transmitted from the therapeutic application 20 as a database. In addition, the management server 1 processes or analyzes various kinds of data stored in the database and provides the results to the therapeutic application 20 or the management application 30.

In addition, the therapeutic application 20 has a function of receiving, from the management server 1, various kinds of data such as advice or countermeasures from medical professionals provided to the management server 1 by the management application 30. Regarding this function, the management server 1 receives various kinds of data such as advice or countermeasures from medical professionals input from the management application 30, and stores the data as a database. Then, the management server 1 provides the various kinds of data stored in the database to the therapeutic application 20 at an appropriate timing.

The management application 30 can access the management server 1 to acquire necessary information. For example, the management application 30 can acquire, through a predetermined management screen or the like, processing results or analysis results of various kinds of data stored in the database of the management server 1. By checking these processing results or analysis results through the management application 30, the medical professional can grasp the patient's medical condition, and input information such as advice or countermeasures for improving the medical condition and transmit the information to the management server 1.

Note that the processing described herein is merely an example. The contents of various kinds of data transmitted from the therapeutic application 20 to the management server 1, the contents of processing executed by the management server 1, and the contents of various kinds of data transmitted from the management application 30 to the management server 1 vary depending on the disease to be treated. Since these contents are not the subject of the invention, detailed description thereof will be omitted.

When the management application 30 is configured as a web application, the therapeutic application 20 and the management application 30 transmit and receive various kinds of information through an operation server connected to the communication network 100, and perform the various processes described above in order to improve the patient's symptoms. In this case, the database for storing various kinds of data may be provided in the operation server, or may be provided as an external storage.

As described above, in order for the therapeutic application 20 and the management application 30 to operate in cooperation through the management server 1 (or the operation server), it is necessary to register, in the management server 1 (or the operation server), the therapeutic application 20 prescribed to the patient on the medical institution terminal 3 and the management application 30 used at the medical institution terminal 3 in association with each other. By performing this association registration, the therapeutic application 20 is activated, so that the therapeutic application 20 can transmit and receive information to and from the management application 30. Details of processing related to this activation will be described below.

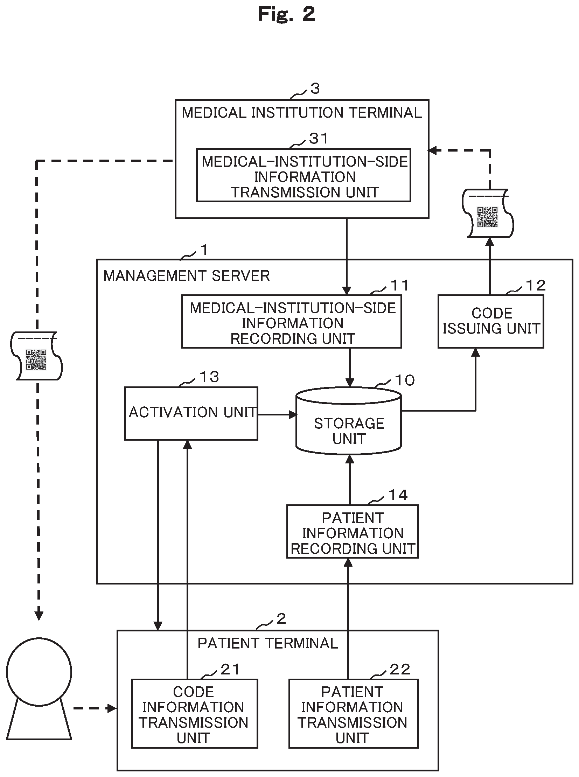

FIG. 2 is a block diagram showing an example of the functional configuration of the management server 1, the patient terminal 2, and the medical institution terminal 3. As shown in FIG. 2, the management server 1 includes, as its functional components, a medical-institution-side information recording unit 11, a code issuing unit 12, an activation unit 13, and a patient information recording unit 14. The patient terminal 2 includes, as its functional components, a code information transmission unit 21 and a patient information transmission unit 22. The medical institution terminal 3 includes a medical-institution-side information transmission unit 31 as its functional component.

The functional blocks 11 to 14 of the management server 1 execute processing described below through cooperation between hardware and software. For example, the processing of the above functional blocks 11 to 14 is executed by the operation of programs stored in storage media, such as a RAM, a ROM, a hard disk, or a semiconductor memory, under the control of a microcomputer configured with a CPU, a RAM, a ROM, and the like. In addition to the microcomputer, a DSP (Digital Signal Processor) may be provided.

The functional blocks 21 and 22 of the patient terminal 2 also execute processing described below through cooperation between hardware and software. For example, the processing of the functional blocks 21 and 22 is executed by the operation of a program of the therapeutic application 20 stored in a storage medium, such as a RAM, a ROM, a hard disk, or a semiconductor memory, under the control of a microcomputer configured with a CPU, a RAM, a ROM, and the like.

The functional block 31 of the medical institution terminal 3 also executes processing described below through cooperation between hardware and software. For example, the processing of the functional block 31 is executed by the operation of a program of the management application 30 stored in a storage medium, such as a RAM, a ROM, a hard disk, or a semiconductor memory, under the control of a microcomputer configured with a CPU, a RAM, a ROM, and the like.

The medical-institution-side information transmission unit 31 of the medical institution terminal 3 transmits medical institution information, which is input through the function of the management application 30, to the management server 1. For example, an information input screen is displayed by the management application 30, and the medical institution information input by a medical professional through the information input screen is transmitted to the management server 1. The medical institution information may be any information that can identify an individual medical institution, and for example, information indicating the name of the medical institution may be used.

When transmitting the medical institution information to the management server 1, the medical-institution-side information transmission unit 31 also transmits identification information that has been assigned in advance to the management application 30 (hereinafter, referred to as a management application ID). The management application ID is recorded in the management application 30, or recorded in the storage unit of the medical institution terminal 3 in association with the management application 30, and the medical institution terminal 3 transmits the medical institution information to the management server 1 together with this management application ID.

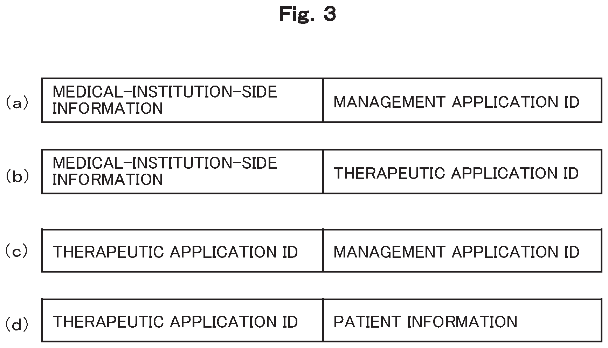

The medical-institution-side information recording unit 11 of the management server 1 receives the medical institution information and the management application ID from the medical institution terminal 3, and records the medical institution information and the management application ID in a storage unit 10 in association with each other, as shown in (a) of FIG. 3. FIG. 3 is a diagram showing an example of information stored in the storage unit 10. Note that FIG. 3 schematically shows the relationship between pieces of information for the purpose of explanation, and the information does not necessarily have to be recorded in the format or form shown in FIG. 3.

The code issuing unit 12 of the management server 1 issues a code, such as a barcode or a two-dimensional code, using the medical institution information recorded in the storage unit 10. For example, the code issuing unit 12 generates print data to print a code recording the medical institution information on paper, and supplies the print data to a printer to issue a code printed on paper (hereinafter, referred to as code paper). Here, the code issuing unit 12 issues a predetermined number of sheets of code paper. The issued code paper is delivered to the medical institution.

Note that the code issuing unit 12 may issue a code (hereinafter, referred to as code data) in the form of the above-described print data or original data for generating the print data. The code data issued by the code issuing unit 12 is provided to, for example, a printing company, and the printing company issues a predetermined number of sheets of code paper. Then, the issued code paper is delivered from the printing company to the medical institution.

At the medical institution, the code paper delivered in the above-described manner is stored. Then, when a decision is made to prescribe the therapeutic application 20 to a visiting patient, the code paper is handed to the patient. The patient who receives the code paper installs the therapeutic application 20 on the patient terminal 2, and reads the code printed on the code paper using a code reading function of the therapeutic application 20.

The code information transmission unit 21 of the patient terminal 2 reads a code using the function of the therapeutic application 20, and transmits the medical institution information obtained by analyzing the code to the management server 1. At this time, the code information transmission unit 21 also transmits identification information (hereinafter, referred to as a therapeutic application ID) that has been assigned in advance to the therapeutic application 20. The therapeutic application ID is recorded in the therapeutic application 20, or recorded in the storage unit of the patient terminal 2 in association with the therapeutic application 20, and the code information transmission unit 21 transmits the medical institution information to the management server 1 together with the therapeutic application ID.

The activation unit 13 of the management server 1 receives the medical institution information and the therapeutic application ID from the patient terminal 2, and records the medical institution information and the therapeutic application ID in the storage unit 10 in association with each other, as shown in (b) of FIG. 3. In addition, the activation unit 13 records, in the storage unit 10, the management application ID (see (a) of FIG. 3) associated with the received medical institution information and the received therapeutic application ID in association with each other as shown in (c) of FIG. 3, thereby activating the therapeutic application 20 into a state in which information can be transmitted and received between the therapeutic application 20 and the management application 30.

That is, by recording the therapeutic application ID and the management application ID in the storage unit 10 in association with each other as shown in (c) of FIG. 3, a state is established in which information can be transmitted and received between the therapeutic application 20 and the management application 30, which are associated with each other, through the management server 1. The activation unit 13 notifies the patient terminal 2 that the activation has been completed.

Note that, although a processing example in which, after information is once recorded in the storage unit 10 as shown in (b) of FIG. 3, the therapeutic application ID and the management application ID are recorded in the storage unit 10 in association with each other as shown in (c) of FIG. 3 has been described herein, recording information as shown in (b) of FIG. 3 can be omitted. In addition, instead of recording information as shown in (a) of FIG. 3 separately from recording information as shown in (c) of FIG. 3, a therapeutic application ID may be added to the information recorded as shown in (a) of FIG. 3 for recording.

After the therapeutic application 20 is activated by the activation unit 13, the patient information transmission unit 22 of the patient terminal 2 transmits, based on the patient's operation, patient information input through the therapeutic application 20 to the management server 1 together with the therapeutic application ID. The patient information input herein is basic information required for using the therapeutic application 20, and examples thereof include the patient's name, sex, age, date of birth, height, and weight. In addition to these, information such as medical history or medication history may also be included. Note that the information listed herein is merely an example and is not limited thereto.

The patient information recording unit 14 of the management server 1 receives the patient information and the therapeutic application ID from the patient terminal 2, and records the patient information in the storage unit 10 in association with the received therapeutic application ID as shown in (d) of FIG. 3. As a result, the therapeutic application ID and the management application ID are recorded in association with each other, and the medical institution information and the patient information are also recorded in association with these IDs.

As described in detail above, according to the present embodiment, the medical institution information transmitted from the medical institution terminal 3 and the management application ID are recorded in the management server 1. The medical treatment application ID that is transmitted together with the medical institution information when the medical institution information, obtained by reading a code generated from the recorded medical institution information with the therapeutic application 20, is transmitted to the management server 1 is associated with the management application ID. As a result, the therapeutic application 20 is activated into a state in which information can be transmitted and received between the therapeutic application 20 and the management application 30.

For this reason, a medical professional does not need to input patient information through the medical institution terminal 3 for each patient to whom the therapeutic application 20 is to be prescribed and transmit the patient information to the management server 1, and it is sufficient for the medical professional to transmit only the medical institution information related to itself to the management server 1 from the medical institution terminal 3. As a result, according to the present embodiment, it is possible to start using the therapeutic application 20 prescribed to a patient in a state associated with the medical institution (management application 30) without imposing a heavy burden on a medical professional.

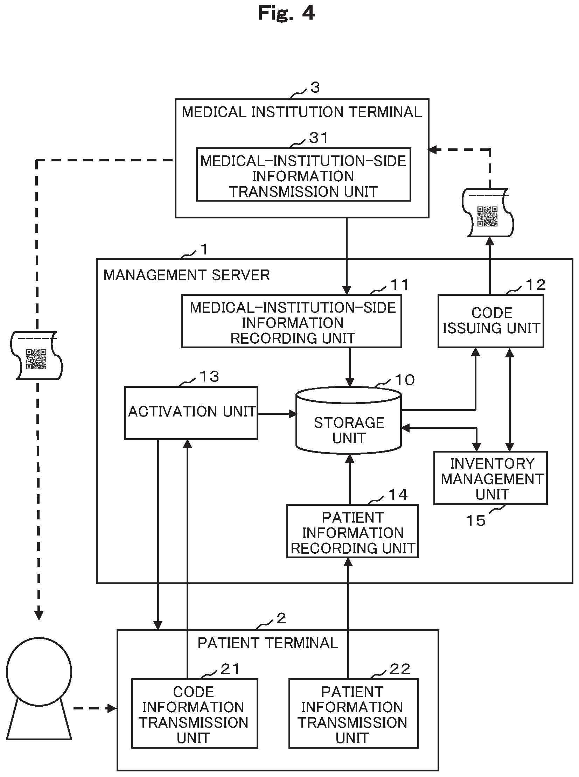

Note that, as shown in FIG. 4, the management server 1 may be made to further have a function of an inventory management unit 15 that manages the inventory of code paper stored at the medical institution, so that additional code paper is automatically issued when the inventory quantity falls below a threshold value.

The inventory management unit 15 stores the number of sheets of code paper issued for each medical institution by the code issuing unit 12 (or the number of sheets of code paper issued by a printing company based on code data issued for each medical institution by the code issuing unit 12). In addition, based on the information stored in the storage unit 10 as shown in FIG. 3, the inventory management unit 15 counts, for each medical institution, the number of therapeutic applications 20 that have been activated (that is, the number of times code paper has been prescribed), and recognizes the inventory quantity of code paper stored at each medical institution based on the difference between these numbers. Then, when the recognized inventory quantity falls below the threshold value, the inventory management unit 15 supplies a code issuance request to the code issuing unit 12. Upon receiving this request, the code issuing unit 12 additionally issues a predetermined number of sheets of code paper.

In this manner, since the medical institution does not need to manage the inventory of code paper, medical professionals are not required to bear the burden associated with inventory management and additional issuance of code paper.

Here, the threshold value that serves as a reference for additional issuance of code paper may be arbitrarily set at the medical institution terminal 3. For example, information on a reference inventory quantity used as a threshold value can be included in the medical institution information transmitted from the medical-institution-side information transmission unit 31 to the management server 1.

Note that, although an example in which the management server 1 or the printing company issues code paper has been described in the above embodiment, the invention is not limited thereto. For example, the code issuing unit 12 may issue a code (hereinafter, referred to as a code image) in the form of image data of a barcode or two-dimensional code, and the code image may be transmitted from the management server 1 to the medical institution terminal 3 through the communication network 100. In this case, the medical institution issues code paper by printing the code image on paper.

In addition, although an example in which code paper is handed over from the medical professional to the patient has been described in the above embodiment, the invention is not limited thereto. For example, as described above, a code image may be transmitted from the management server 1 to the medical institution terminal 3, and the code image may be stored in the medical institution terminal 3. Then, when a medical professional decides to prescribe the therapeutic application 20 to a patient, the code image may be transmitted from the medical institution terminal 3 to the patient terminal 2 through the communication network 100. In this case, the code information transmission unit 21 analyzes the code image, and transmits medical institution information obtained thereby to the management server 1 together with the therapeutic application ID.

In addition, although an example in which a code recording medical institution information is issued has been described in the above embodiment, the invention is not limited thereto. For example, the code issuing unit 12 may issue a code in which the management application ID is recorded. In this case, the code information transmission unit 21 analyzes the code in which the management application ID is recorded, instead of the medical institution information, using the function of the therapeutic application 20, and transmits the management application ID obtained thereby to the management server 1 together with the therapeutic application ID. The activation unit 13 receives the management application ID and the therapeutic application ID from the patient terminal 2 and records the received management application ID and therapeutic application ID in the storage unit 10 in association with each other, thereby activating the therapeutic application 20 so that information can be transmitted and received between the therapeutic application 20 and the management application 30.

In addition, although the configuration in which the management server 1 performs the activation of the therapeutic application 20 and the processing for cooperation with the therapeutic application 20 after activation has been described in the above embodiment, the invention is not limited thereto. A configuration may be adopted in which, separately from the management server 1 that performs the activation of the therapeutic application 20, an operation server that performs processing for cooperation with the therapeutic application 20 after activation is provided. As described above, this operation server may be configured to operate the management application 30 that is configured as a web application. Alternatively, a configuration may be adopted in which the operation server includes the medical-institution-side information recording unit 11, the patient information recording unit 14, and the management application 30 and the management server 1 includes the code issuing unit 12 and the activation unit 13. In this case, the operation server corresponds to the first management server in the claims, and the management server 1 corresponds to the second management server.

In addition, although the configuration in which medical institution information is input using the function of the management application 30 at the medical institution terminal 3 and the medical institution information is transmitted from the medical institution terminal 3 to the management server 1 and recorded therein has been described in the above embodiment, the invention is not limited thereto. For example, a configuration may be adopted in which an external terminal used by an external organization, such as a pharmaceutical company having a relationship with the medical institution, accesses the management application 30 (a web application), inputs medical institution information from the external terminal, and transmits the input medical institution information to the management server 1. In this manner, medical professionals not only do not need to input patient information from the medical institution terminal 3 for each patient to whom the therapeutic application 20 is to be prescribed and transmit the patient information to the management server 1, but also do not need to input medical institution information and transmit the medical institution information to the management server 1. As a result, the burden on medical professionals can be significantly reduced.

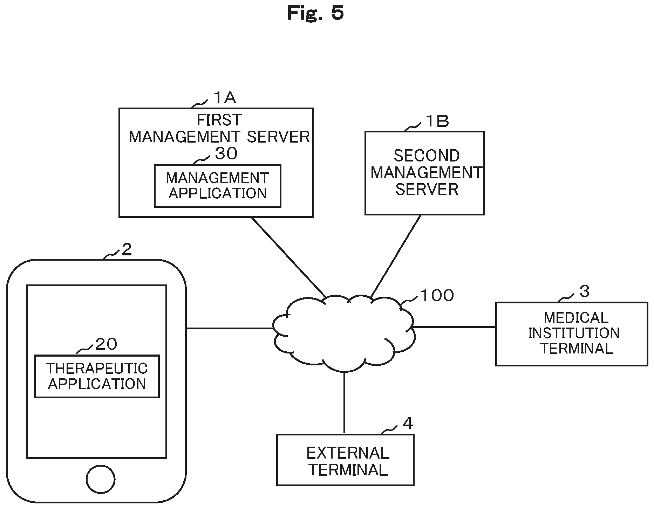

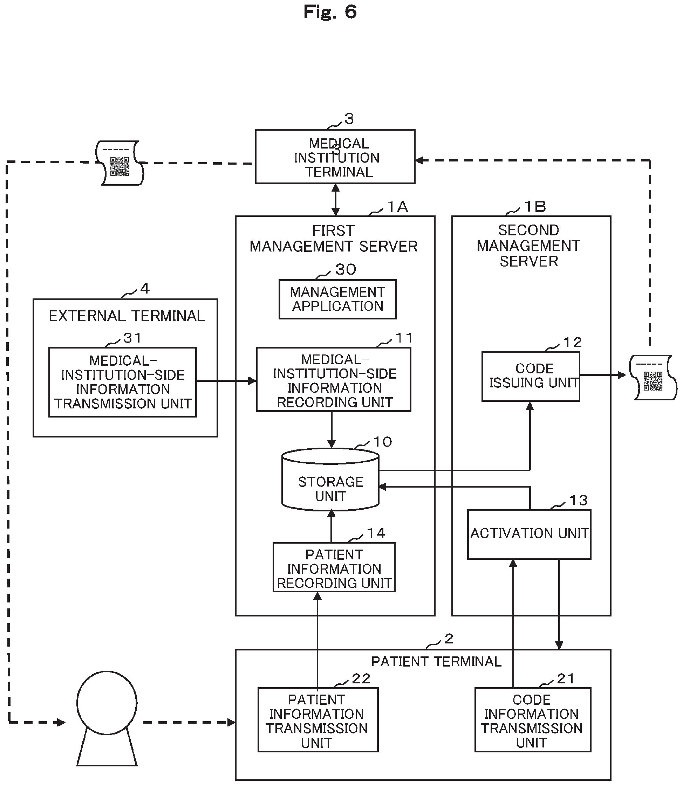

FIGS. 5 and 6 are diagrams showing examples of the configuration of a therapeutic application management system according to a modification example. In this modified example, a first management server 1A (operation server) and a second management server 1B are provided, as well as an external terminal 4 used by an external organization such as a pharmaceutical company. As shown in FIG. 6, the first management server 1A includes the storage unit 10, the medical-institution-side information recording unit 11, the patient information recording unit 14, and the management application 30, while the second management server 1B includes the code issuing unit 12 and the activation unit 13. In addition, the external terminal 4 includes the medical-institution-side information transmission unit 31.

The above embodiments are merely examples of specific implementations for carrying out the invention, and should not be construed as limiting the technical scope of the invention. That is, the invention can be implemented in various forms without departing from the gist or the essential features thereof.

### Reference Signs List

- 1:: management server
- 1A:: first management server (operation server)
- 1B:: second management server
- 2:: patient terminal
- 3:: medical institution terminal
- 4:: external terminal
- 10:: storage unit
- 11:: medical-institution-side information recording unit
- 12:: code issuing unit
- 13:: activation unit
- 14:: patient information recording unit
- 15:: inventory management unit
- 20:: therapeutic application
- 21:: code information transmission unit
- 22:: patient information transmission unit
- 30:: management application
- 31:: medical-institution-side information transmission unit

## Claims

1. A therapeutic application management system, comprising:
a patient terminal on which a therapeutic application is installed; and
a management server that manages the therapeutic application and a management application for transmitting and receiving information in cooperation with the therapeutic application,
wherein the management server includes:
a medical-institution-side information recording unit that records, in a storage unit, medical institution information related to a medical institution using the management application in association with identification information of the management application;
a code issuing unit that issues a code in which the medical institution information is recorded; and
an activation unit that receives the medical institution information and identification information of the therapeutic application from the patient terminal and records, in the storage unit, the identification information of the management application associated with the received medical institution information in association with the received identification information of the therapeutic application, thereby activating the therapeutic application into a state in which information can be transmitted and received between the therapeutic application and the management application, and
the patient terminal includes a code information transmission unit that transmits, to the management server, the medical institution information obtained by analyzing the code recording the medical institution information using a function of the therapeutic application, together with the identification information of the therapeutic application.

2. The therapeutic application management system according to claim 1,
wherein the patient terminal further includes a patient information transmission unit that, after the therapeutic application is activated by the activation unit, transmits patient information input through the therapeutic application and the identification information of the therapeutic application to the management server, and
the management server further includes a patient information recording unit that receives the patient information and the identification information of the therapeutic application from the patient terminal and records the patient information in the storage unit in association with the received identification information of the therapeutic application.

3. The therapeutic application management system according to claim 1 or 2,
wherein the activation unit receives the identification information of the management application and the identification information of the therapeutic application from the patient terminal, instead of receiving the medical institution information and the identification information of the therapeutic application from the patient terminal, and records the received identification information of the management application and the received identification information of the therapeutic application in the storage unit in association with each other, thereby activating the therapeutic application into a state in which information can be transmitted and received between the therapeutic application and the management application, and
the code information transmission unit analyzes, using a function of the therapeutic application, a code in which identification information of the management application is recorded instead of the medical institution information, and transmits the identification information of the management application obtained by the analysis to the management server together with the identification information of the therapeutic application.

4. The therapeutic application management system according to claim 1,
wherein the management server further includes an inventory management unit that manages an inventory quantity of codes at a medical institution based on the number of codes issued by the code issuing unit and the number of therapeutic applications activated by the activation unit, and
the code issuing unit issues the code when the inventory quantity managed by the inventory management unit falls below a threshold value.

5. The therapeutic application management system according to claim 1,
wherein the management server includes:
a first management server including the medical-institution-side information recording unit; and
a second management server including the code issuing unit and the activation unit.

6. A management server for managing a therapeutic application installed on a patient terminal and a management application for transmitting and receiving information in cooperation with the therapeutic application, the management server comprising:
a code issuing unit that issues a code in which medical institution information related to a medical institution that uses the management application is recorded; and
an activation unit that receives, from the patient terminal, the medical institution information obtained by analyzing the code recording the medical institution information using a function of the therapeutic application and identification information of the therapeutic application and records identification information of the management application, which is stored in a storage unit in association with the received medical institution information, in the storage unit in association with the received identification information of the therapeutic application, thereby activating the therapeutic application into a state in which information can be transmitted and received between the therapeutic application and the management application.
